Europäisches Patentamt

European Patent Office

Office européen des brevets

(11) Publication number: **0 254 368**
**B1**

(12) **EUROPEAN PATENT SPECIFICATION**

(45) Date of publication of patent specification: **10.10.90**

(51) Int. Cl.⁵: **C 07 C 309/02, C 07 C 303/04**

(21) Application number: **87201364.4**

(22) Date of filing: **17.07.87**

(54) Process for extracting paraffins from their mixtures with alkanesulphonic acids.

(30) Priority: **23.07.86 IT 2122586**

(43) Date of publication of application:
**27.01.88 Bulletin 88/04**

(45) Publication of the grant of the patent:
**10.10.90 Bulletin 90/41**

(84) Designated Contracting States:
**AT BE CH DE ES FR GB GR LI LU NL SE**

(56) References cited:
**EP-A-0 239 177**
**FR-A-2 376 842**
**US-A- 228 598**

(73) Proprietor: **ENIRICERCHE S.p.A.**
**Corso Venezia 16**
**I-20121 Milan (IT)**

(73) Proprietor: **ENICHEM AUGUSTA S.p.A.**
**Via Ruggero Settimo 55**
**I-90139 Palermo (IT)**

(72) Inventor: **Franco, Cosimo**
**Via Bari 3**
**I-89044 Locri Reggio Calabria (IT)**
Inventor: **Carrillo, Gerardo**
**Via Morandi 26**
**I-20097 San Donato Milanese Milan (IT)**
Inventor: **Faggian, Lucio**
**Via Kennedy 32**
**I-20097 San Donato Milanese Milan (IT)**

(74) Representative: **Roggero, Sergio et al**
**Ing. Barzanò & Zanardo Milano S.p.A. Via**
**Borgonuovo 10**
**I-20121 Milano (IT)**

Courier Press, Leamington Spa, England.

## Description

This invention relates to a process for extracting paraffins from their mixtures with alkanesulphonic acids. In the description the term "alkanesulphonic acid" is synonymous with paraffinsulphonic acid.

Alkanesulphonic acids containing between 12 and 18 carbon atoms are generally prepared by sulphoxidation of $C_{12}$—$C_{18}$ n-paraffins with $SO_2$ and $O_2$ using UV radiation for reaction initiation.

The reaction product obtained from the sulphoxidation reactor consists of a mixture containing small percentages of alkanesulphonic acids, water and sulphuric acid, but mostly unreacted n-paraffins.

Most of the paraffins can be easily separated from said mixture, but a substantial fraction of them remains together with the sulphuric acid, the water and the alkanesulphonic acids. It is important to note that the paraffins must be separated to the maximum possible extent not only for obvious economic reasons, but also because their presence in alkanesulphonic acids is undesirable.

The known art gives suggestions for separating said paraffins from the rest of the sulphuric acid, alkanesulphonic acid and water mixture, one of these suggestions being contained in European patent application 131913, in particular in Example 1, according to which the mixture containing alkanesulphonic acids, unreacted paraffins, water and sulphuric acid is treated with isopropanol in a quantity of 15%, to separate the mixture into three distinct phases, the upper one essentially consisting of paraffins, the lower one consisting of water, sulphuric acid and isopropanol, and the intermediate one containing alkanesulphonic acids, sulphuric acid, water, paraffins and isopropanol. The intermediate phase is then mixed with methylene chloride to separate an aqueous sulphuric acid phase containing isopropanol and a little methylene chloride from a phase containing alkanesulphonic acids, paraffins, water, methylene chloride and sulphuric acid, this being neutralised with soda and concentrated to a temperature of up to 200 degrees centigrade to separate the paraffins.

This procedure for removing the paraffins is obviously complicated, and notwithstanding its various extraction stages it is still necessary to use high-temperature treatment at the end, which in all cases damages the product obtained.

With the known process it is therefore not possible to prepare free alkanesulphonic acids or their salts with weak bases, as these are unstable at high temperature.

It has been surprisingly found that the previously described drawbacks of the known art regarding the separation of n-paraffins can be obviated in a very simple manner by mixing the mixture originating from the n-paraffin sulphoxidation reactor, after removing its dissolved sulphur dioxide by known means, with limited quantities of alcohols containing four or less than four carbon atoms, until a two-phase mixture is formed, and then extracting the two-phase mixture which supercritical $CO_2$.

The present invention provides a process for extracting $C_{12}$—$C_{18}$ paraffins from their mixtures with $C_{12}$—$C_{18}$ alkanesulphonic acids, comprising after previously removing excess $SO_2$, decanting the reaction product originating from a region of $C_{12}$—$C_{18}$ n-paraffin sulphoxidation by $SO_2$ and $O_2$ in the presence of UV radiation and water at a temperature of between 25 and 50 degrees centigrade in order to naturally separate most of the unreacted n-paraffins from the remainder of the reaction mixture, and mixing said remainder of the reaction mixture with an aliphatic alcohol containing four or less than four carbon atoms, preferably isopropanol, characterised in that the aliphatic alcohol is used in the quantity necessary to form a two-phase mixture, and the two-phase mixture is extracted with carbon dioxide under supercritical conditions, so separating the paraffins from the alkanesulphonic acids and from the suphuric acid and the water.

The resultant mixture can then be conventionally neutralised, to obtain alkanesulphonates of the desired type.

If desired, the sulphuric acid can be separated from the paraffin-free alkanesulphonates by known methods, such as mixing with suitable substances or precipitation to form insoluble salts.

The alcohol used is preferably isopropanol, and the quantity of alcohol used to form the two-phase mixture according to the invention is such that its concentration therein ranges from 3 to 8.5% by weight, and is preferably about 5% by weight.

The conditions under which extraction with supercritical $CO_2$ is carried out are a temperature of between 32 and 80 degrees centigrade, a pressure of between 75 and 350 bar, and a weight ratio of $CO_2$ used for extraction to alkanesulphonic acids of between 1:1 and 50:1.

Some examples are given hereinafter in order to better illustrate the invention, but without intending to limit it thereto or thereby.

## Example 1

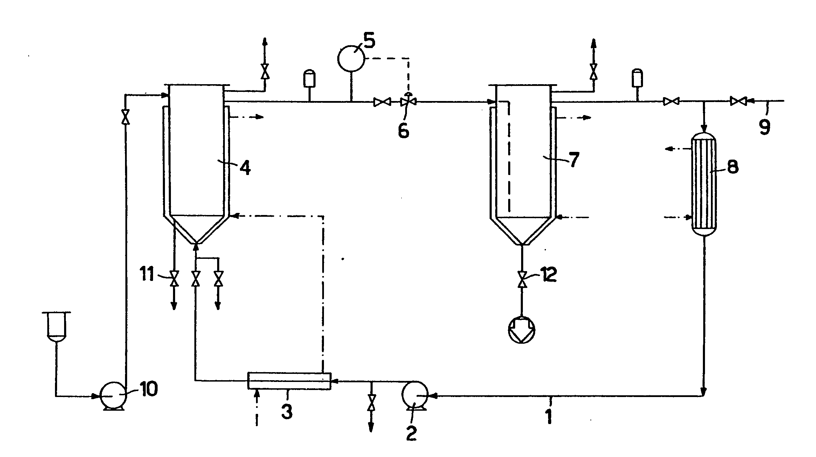

The laboratory extraction apparatus shown diagrammatically in the Figure was used.

It consists of a refrigeration cycle for condensing $CO_2$ in the heat exchanger 8. The liquid $CO_2$ 1 is pumped by the diaphragm pump 2 to the preheater 3 and then to the extractor 4. The temperature of 3 and 4 is maintained constant and at the same value by circulating water from a temperature-controlled bath. The pressure in 4 is kept constant at the required value by the controller 5 and control valve 6.

The $CO_2$ containing the products extracted from the crude mixture fed into 4 passes through 6 and leaves the supercritical field in the separator 7, where the $CO_2$ evaporates and is condensed in 8 to then return to the already described cycle, whereas the extract remains in the separator 7. Any required make-up $CO_2$ is fed through 9.

EP 0 254 368 B1

The separator 7 is provided with two diametrically opposite sight glasses for visually checking the level.

This is kept constant by adjusting the temperature of the water originating from a second temperature-controlled bath. The pressure in 7 is kept constant by a pressure switch which operates the refrigeration cycle.

The extractor is filled with stainless steel packing held down by a demister.

A second pump 10 is used for continuous operation to feed the crude product to be extracted. In this case, the refined product is discharged through the valve 11.

124.3 g of crude mixture (from which decantable n-paraffins and $SO_2$ have been removed) containing:

| | |
|---|---|
| $C_{12}$—$C_{18}$ alkanesulphonic acids | 24.74% by weight |
| $C_{12}$—$C_{18}$ n-paraffins | 26.46% by weight |
| water | 40.94% by weight |
| sulphuric acid | 7.86% by weight |

were fed into the extractor 4, together with 6.4 g of isopropyl alcohol.

The extractor 4 was temperature-controlled at 45 degrees centigrade, and $CO_2$ was then fed in at a throughput of 1.46 kg/h, maintaining the pressure in the extractor at 200 bar.

After 1 hour of extraction, $CO_2$ feed was suspended and the product contained in the extractor was discharged and analysed. The paraffin quantity extracted was found to be 96% of that present in the crude product feed.

Example 2 (Comparative, without isopropanol addition)

123.9 g of a crude alkanesulphonic acid mixture having the same composition as in Example 1 were extracted with supercritical $CO_2$ under the conditions and for the time stated in Example 1.

Analysis of the refined product showed that the paraffins had been extracted to an extent of 67.5% of the quantity present in the crude product feed.

**Claims**

1. A process for extracting $C_{12}$—$C_{18}$ paraffins from their mixtures with alkanesulphonic $C_{12}$—$C_{18}$ acids where the mixtures have been obtained by sulphoxidation of n-paraffins with $SO_2$ and $O_2$ in the presence of UV radiation and water at a temperature of between 25 and 50 degrees centigrade and the reaction product obtained has been freed of excess $SO_2$ and subjected to decantation in order to naturally separate most of the unreacted n-paraffins from the remainder of the reaction mixture, the remainder of the reaction mixture having been mixed with an aliphatic alcohol containing four or less than four carbon atoms, said alcohol being preferably isopropanol, characterised in that the aliphatic alcohol is used in the quantity necessary to form a two-phase mixture, and the two-phase mixture is extracted with supercritical $CO_2$, so separating the paraffins from the alkanesulphonic acid mixture.

2. A process as claimed in claim 1, characterised in that the alcohol quantity required to form the two-phase mixture is such as to obtain a concentration in the mixture of between 3% and 8.5% by weight.

3. A process as claimed in claim 2, characterised in that the mixture has an alcohol concentration of about 5% by weight.

4. A process as claimed in claim 1, characterised in that the conditions under which extraction with supercritical $CO_2$ is carried out are a temperature of between 32 and 80 degrees centigrade and a pressure of between 75 and 350 bar.

5. A process as claimed in claim 1, characterised in that the weight ratio of $CO_2$ used for extraction to alkanesulphonic acids is between 1:1 and 50:1.

6. A process as claimed in claim 1, characterised in that the alkanesulphonic acid mixture freed of paraffins is conventionally neutralised to prepare the corresponding alkanesulphonates.

**Patentansprüche**

1. Verfahren zum Extrahieren von $C_{12}$—$C_{18}$-Paraffinen aus ihren Gemischen mit $C_{12}$—$C_{18}$-Alkansulfonsäuren, worin die Gemische durch Sulfoxidation von n-Paraffinen mit $SO_2$ und $O_2$ in Gegenwart von UV-Strahlung und Wasser bei einer Temperatur von 25 bis 50°C erhalten worden sind, und das erhaltene Reaktionsgemisch von überschüssigem $SO_2$ befreit worden ist und dekantiert worden ist, um auf natürlichem Wege den Hauptteil der nicht-umgesetzten Paraffine vom restlichen Reaktionsgemisch abzutrennen, wobei das restliche Reaktionsgemisch mit einem vier oder weniger Kohlenstoffatome enthaltenden aliphatischen Alkohol vermischt worden ist, welcher Alkohol vorzugsweise Isopropanol ist, dadurch gekennzeichnet, daß der aliphatische Alkohol in der zur Ausbildung eines Zweiphasengemisches erforderlichen Menge verwendet wird und das Zweiphasengemisch mit überkritischem $CO_2$ extrahiert wird und solcherart die Paraffine aus dem Alkansulfonsäurengemisch abgetrennt werden.

2. Verfahren nach Anspruch 1, dadurch gekennzeichnet, daß die zur Ausbildung des

3

Zweiphasengemisches erforderliche Alkoholmenge so groß ist, daß eine Konzentration in dem Gemisch von 3 bis 8,5 Gew.-% erreicht wird.

3. Verfahren nach Anspruch 2, dadurch gekennzeichnet, daß das Gemisch eine Alkoholkonzentration von etwa 5 Gew.-% aufweist.

4. Verfahren nach Anspruch 1, dadurch gekennzeichnet, daß die Bedingungen, unter denen die Extraktion mit überkritischem $CO_2$ ausgeführt wird, eine Temperatur von 32 bis 80°C und einen Druck von 75 bis 350 bar sind.

5. Verfahren nach Anspruch 1, dadurch gekennzeichnet, daß das Gewichtsverhältnis von zur Extraktion verwendeten Kohlendioxid zu Alkansulfonsäuren von 1:1 bis 50:1 beträgt.

6. Verfahren nach Anspruch 1, dadurch gekennzeichnet, daß das von Paraffinen befreite Alkansulfonsäurengemisch in üblicher Weise zur Ausbildung der entsprechenden Alkansulfonate neutralisiert wird.

**Revendications**

1. Procédé pour extraire des paraffines en $C_{12}$—$C_{18}$ de leurs mélanges avec des acides alcane-sulfoniques en $C_{12}$—$C_{18}$, ces mélanges ayant été obtenus dans la sulfoxydation de n-paraffines à l'aide de $SO_2$ et de $O_2$, en présence de rayonnement UV et d'eau, à une température située entre 25 et 50°C, le produit de réaction obtenu ayant été débarrassé de l'excès de $SO_2$ et soumis à une décantation dans le but de séparer naturellement la majeure partie des n-paraffines n'ayant pas réagi d'avec le reste du mélange réactionnel, le reste du mélange réactionnel ayant été mélangé avec un alcool aliphatique contenant au plus quatre atomes de carbone, ledit alcool étant de préférence l'isopropanol, caractérisé en ce que l'alcool aliphatique est utilisé en la quantité nécessaire pour former un mélange biphasique, et en ce que le mélange biphasique est extrait avec du $CO_2$ à l'état supercritique, ce qui permet de séparer les paraffines d'avec le mélange d'acides alcane-sulfoniques.

2. Procédé tel que revendiqué dans la revendication 1, caractérisé en ce que la quantité d'alcool nécessaire pour former le mélange biphasique est telle que l'on en obtient, dans le mélange, une concentration située entre 3% et 8,5% en poids.

3. Procédé tel que revendiqué dans la revendication 2, caractérisé en ce que le mélange présente une concentration d'alcool d'environ 5% en poids.

4. Procédé tel que revendiqué dans la revendication 1, caractérisé en ce que les conditions dans lesquelles est effectuée l'extraction avec $CO_2$ à l'état supercritique sont une température située entre 32 et 80°C et une pression située entre 75 et 350 bars.

5. Procédé tel que revendiqué dans la revendication 1, caractérisé en ce que le rapport pondéral du $CO_2$ utilisé pour l'extraction aux acides alcane-sulfoniques vaut entre 1:1 et 50:1.

6. Procédé tel que revendiqué dans la revendication 1, caractérisé en ce que le mélange d'acides alcanesulfoniques débarrassé des paraffines est neutralisé de façon classique, dans le but de préparer les alcane-sulfonates correspondants.